(19) Europäisches Patentamt
European Patent Office
Office européen des brevets

(11) EP 0 839 782 B1

(12) # EUROPEAN PATENT SPECIFICATION

(45) Date of publication and mention of the grant of the patent:
**05.04.2000 Bulletin 2000/14**

(51) Int Cl.[7]: **C07C 4/06**, C10G 9/16, C10G 75/04

(21) Application number: **96307868.8**

(22) Date of filing: **30.10.1996**

(54) **Process for the inhibition of coke formation in pyrolysis furnaces**

Verfahren zur Hemmung von Verkoken in Pyrolyse-Ofen

Procédé pour l'inhibition de la formation de coke dans les fours de pyrolyse

(84) Designated Contracting States:
**BE DE DK ES FI FR GB IT NL**

(43) Date of publication of application:
**06.05.1998 Bulletin 1998/19**

(73) Proprietor: **NALCO/EXXON ENERGY CHEMICALS, L.P.**
**Sugarland, TX 77478 (US)**

(72) Inventors:
- **Tong, Youdong**
  **1510 Houston, Texas (US)**
- **Poindexter, Michael K.**
  **Sugar Land, Texas 77478 (US)**
- **Rowe, Tom C.**
  **Missouri City, Texas 77459 (US)**

(74) Representative:
**Harrison, David Christopher et al**
**MEWBURN ELLIS**
**York House**
**23 Kingsway**
**London WC2B 6HP (GB)**

(56) References cited:
**US-A- 3 437 583**
**US-A- 3 574 088**
**US-A- 4 440 625**
**US-A- 4 456 526**
**US-A- 5 221 461**
**US-A- 5 271 824**
**US-A- 5 460 712**

- **PATENT ABSTRACTS OF JAPAN vol. 010, no. 373 (C-391), 12 December 1986 & JP 61 166882 A (HAKUTOU KAGAKU KK), 28 July 1986,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 839 782 B1

## Description

The invention relates to the production of ethylene and similar products by steam pyrolysis of hydrocarbon feedstocks in a pyrolysis furnace and, more particularly, to the inhibition of coke deposits on the surfaces of the radiant heating section of the furnace and surfaces immediately downstream from such sections in contact with the hydrocarbon feedstock and by-products of the pyrolysis reactions during the processing of the hydrocarbon feedstock.

Ethylene manufacture entails the use of pyrolysis furnaces (also known as steam crackers or ethylene furnaces) to thermally crack various gaseous and liquid petroleum feedstocks to manufacture ethylene as well as other useful products. Typical gaseous feedstocks include ethane, propane, butane, and mixtures thereof. Typical liquid feedstocks include naphtha, kerosene, gas oil, and mixtures thereof.

The hydrocarbon feedstocks are cracked in the tube reactors of a pyrolysis furnace typically at temperatures ranging from 700 to 1100°C. Steam is generally used in the cracking reactions to control undesired reactions or processes, such as coke formation. The hydrocarbon feedstocks and the steam are mixed and preheated when they pass through the convection section of the pyrolysis furnace. The cracking reactions of the hydrocarbon feedstocks occur in the radiant section of the pyrolysis furnace. The cracked product effluent from the radiant section is quenched through transfer line exchangers (TLEs) and oil and/or water quench towers, and then it is fractionated and purified in the downstream processes to desired products. In general, ethylene is the major and the most desired of the products.

Metal alloys containing high nickel, iron, and chromium are widely used in industry as the construction materials for the furnace tubes to withstand the high temperature and extreme operating environments. However, nickel and iron are also well known catalysts for the reactions leading to the formation of coke deposition.

Coke deposits are by-products of the cracking reactions. Even though the reactions leading to coke deposition are not significant relative to those that produce the desired products, the amount of the coke formed is enough to make coke deposition a major limitation for the pyrolysis furnace operation. Fouling of the furnace reactor coils and TLEs occurs because of coke deposition. Coke deposition decreases the effective cross-sectional area of the process stream, which increases the pressure drop across the furnace reactors and TLEs. The pressure buildup in the reactor adversely affects the yield of ethylene.

Additionally, since coke is a good thermal insulator, the buildup of coke on the inside surface of the reactor wall requires a gradual increase in furnace firing to ensure adequate heat transfer to maintain the desired conversion level. Eventually, tube skin temperature will reach the limit of the metal alloy, and lower conversion will be the consequence of insufficient heat flux. Also, higher metal tube temperatures accelerate reactor tube deterioration and shorten the tube life.

Depending on coke deposition rate, the cracking operations must be periodically terminated or shut down for cleaning (i.e., decoking). Cleaning operations are carried out either mechanically or by passing steam and/or air through the coils and TLEs to burn off the coke buildup. In addition to the periodic cleaning, crash shutdowns are sometimes required because of dangerous situations resulting from coke buildup in furnace reactor coils or TLEs. Run length, which is the operation time between the cleanings, averages from one week to four months depending in part upon the rate of fouling of the furnace reactor coils and TLEs. Any process improvement or chemical treatment that could reduce coke deposition and increase run length would obviously lead to higher production capacity, fewer days lost to cleaning, and lower operation and maintenance costs.

Extensive research has been carried out to understand the mechanisms of coke formation and to search for solutions of eliminating the coke deposition. Concerning the reactions contributing to coke formation, coke can be generally classified into two major categories: catalytic and non-catalytic coke. The reactions catalyzed by metals, such as dehydrogenation reactions, are the origins of the catalytic coke, while the non-catalytic coke is the product of certain interface radical reactions. In both cases, a physical contact between gas phase coke precursors and surface active sites is necessary. Thus, elimination of this physical contact will significantly lower the overall coke formation and deposition. One method to prevent this physical contact would be to build an effective, catalytically inactive physical barrier which would isolate gas phase coke precursors from active surface sites.

Chemical additives containing phosphorus, sulfur, aluminum, silicon, tin, bismuth, hydrocarbons, alkali and alkali earth metals, rare earth metals, and the like have been studied or used as coke inhibitors to reduce coke formation and deposition. It is believed that some of the coke inhibitors, such as those containing aluminum, silicon, and hydrocarbons, are based on the principle of generating a catalytically inactive physical barrier which isolates gas phase coke precursors from active surface sites. Of all the choices, hydrocarbons are more attractive because their use does not introduce any foreign elements into the process, as foreign elements in general raise concerns about their potential side effects.

It is known that coke may be catalytically active, promoting further coke formation, or catalytically inactive, inhibiting or reducing the rate of coke formation. The formation of catalytically active coke results in an auto-acceleration of the coking rate, while the formation of catalytically inactive coke results in at least a de-acceleration of the coking rate. The formation of the different cokes depends upon the hydrocarbon feedstock used in the pyrolysis process. A

catalytically active coke is formed when acetylene is used as a feedstock. Such coke acts as a catalyst for further coke formation. Catalytically inactive coke is formed when a butadiene or benzene feedstock is used in a pyrolysis process. Such catalytically inactive coke reduced the coke formation rate. Catalytically active coke has been found to contain a considerable amount of metal granules. Therefore, it would be desirable to generate a layer of catalytically inactive coke, as such a coke layer may serve the purpose of a physical barrier to isolate the coke precursors in the hydrocarbon feedstock and pyrolysis by-products from contacting active surface sites.

Leftin et al., US-A-4,176,045, teaches a method to minimize coke deposition in the production of lower olefins by co-cracking a low-coking hydrocarbon with a feedstock having a high-coking tendency. It claimed that such blending would result in a low-coking hydrocarbon feedstock if a proper blending ratio is chosen. The coking tendency is determined by the Coking Inhibition Index which depends on specific gravity, sulfur content, and aromaticity. The Leftin reference did not address the issues concerning the fouling of the convection section of the furnace and the TLEs as well as any downstream operation when co-processing two hydrocarbons which differ in gravity, sulfur content, and aromaticity. In a later publication, Leftin and Newsome attributed the inhibition potential of the low-coking hydrocarbons to their ability to form an amorphous coke deposit which encapsulates catalytically active metal sites.

Bach et al., DD-A-222,324, teaches a method of reducing carbon-rich solid deposits in the pyrolysis reactor without additional coke formation in the condensation and cooling system by adding between 1 and 30% toluene or toluene-containing fractions (at the C7 portion of naphtha) to feedstocks from ethane up to Vacuum Gas Oil (VGO). A reduction in coking by as much as 50% was reported, and in addition, an increase in the target products such as benzene, xylene, and styrene was obtained.

Buddell et al., US-A-4,599,480, teaches a method in which two cracking feedstocks are used in a sequence. The first feedstock is selected from naphtha or gasoline boiling range or C3-C12 paraffin hydrocarbons. The second feedstock uses a lower paraffin than the paraffin used in the first feedstock. The first fcedstock is cracked to place an amorphous relatively smooth layer of coke on interior walls of the thermal cracking tubes. To achieve a required thickness of this coke layer (between 1/16 and 1/8 inch), the first feedstock has to be on stream for a time as long as 11 days before operations can be switched to the second feedstock. Buddell did not address the impact of this sequential cracking of two different feedstocks on furnace operation, the fouling tendency in convection section of the furnace or the TLEs due to processing heavier feedstocks in the first cracking operation, and the adhesive property of the coke layer formed during the cracking of two different feedstocks.

Aromatics are well-known precursors for tar or coke formation. The higher coke formation tendency of certain heavy hydrocarbon feeds, such as gas oils, has been attributed to their higher aromatic content. In the case of cracking paraffinic materials, it is proposed that aromatics are generated through the cyclization of ethylene or propylene with higher di-olefins or the reactions of olefins with alkyl-type radicals. It would follow that aromatics are part of the least desirable components in a hydrocarbon feedstock because they are coke precursors, and as such, are of high coking tendency. It is least desirable to process a feedstock of a high aromatic content under a conventional cracking condition with respect to coke deposition.

A method could exist by which a catalytically inactive coke layer is formed on the surface of the radiant heating section and the surfaces immediately downstream from the radiant heating section that are in contact with a hydrocarbon feedstock and/or pyrolysis products during the processing of the hydrocarbon feedstock. The catalytically inactive coke layer, as an effective physical barrier between the coke precursors and active surface sites, would reduce the rate of coke formation, thereby, increasing run length and production levels of desired product. Ideally, this catalytically inactive coke layer could be formed using an effective amount of a coke inhibiting compound within an acceptable time, and more importantly, the formation of this layer would not generate any adverse side effects. In addition, the catalytically inactive coke layer could be formed without the addition of foreign elements which may result in additional concerns or detrimental side effects.

The invention is a method for inhibiting the formation of coke on the surfaces of the radiant heating sections in pyrolysis furnaces and the surfaces immediately downstream from such sections (e.g. TLEs) in contact with a hydrocarbon feedstock during the processing of the feedstock. The present invention is a method for inhibiting the formation of coke on the surfaces of a radiant heating section of a pyrolysis furnace and the surfaces immediately downstream of such section in contact with a hydrocarbon feedstock which comprises decoking the pyrolysis furnace and prior to processing the hydrocarbon feedstock, adding an inhibiting compound to the pyrolysis furnace. The inhibiting compound is selected from the group consisting of substituted benzenes, substituted naphthalenes, substituted anthracenes, substituted phenanthrenes, and mixtures thereof wherein the inhibiting compound contains at least one substitutent having at least 2 carbon atoms.

A thin catalytically inactive coke layer is formed on the surfaces of the pyrolysis furnace. The hydrocarbon feedstock is then fed into the furnace, whereby the surfaces of the furnace are inhibited against the formation of a catalytically active coke during the processing of the hydrocarbon feedstock.

The addition of the inhibiting compound is usually started prior to the processing of a hydrocarbon feedstock, and may be continued during the processing of a hydrocarbon feedstock. The addition of the inhibiting compound may

be discontinued prior to or during the processing of the hydrocarbon feedstock, or it may be started during the processing of the hydrocarbon feedstock. The inhibiting compound may be added on a continuous or intermittent basis before or during the processing of a hydrocarbon feedstock.

## Brief Description of the Drawings

FIG. 1 shows the coke buildup and the corresponding coking rate with time on stream recorded by an electrobalance during a blank run.

FIG. 2 is a coking rate vs. time on stream plot illustrating the anti-coking performance of additive B vs. the blank.

## Description of the Invention

The invention is a method for inhibiting the formation of coke on the surfaces of the radiant heating sections in pyrolysis furnaces and the surfaces immediately downstream from such sections (e.g. TLEs) in contact with a hydrocarbon feedstock during the processing of the feedstock. The term hydrocarbon feedstock, as used herein, is understood to also include the products of the pyrolysis reactions. The method for inhibiting the formation of coke on the surfaces of a radiant heating section of a pyrolysis furnace and the surfaces immediately downstream of such section in contact with a hydrocarbon feedstock comprises decoking the pyrolysis furnace and prior to processing the hydrocarbon feedstock, adding an inhibiting compound to the pyrolysis furnace. The inhibiting compound is selected from the group consisting of substituted benzenes, substituted naphthalenes, substituted anthracenes, substituted phenanthrenes, and mixtures thereof wherein the inhibiting compound contains at least one substitutent having at least 2 carbon atoms.

A thin catalytically inactive coke layer is formed on the surfaces of the pyrolysis furnace. The thickness of the coke layer can range from about a molecular thickness to a level of coke formation that does not substantially restrict the flow of the hydrocarbon feedstock through the pyrolysis furnace. The hydrocarbon feedstock is then fed into the furnace, whereby the surfaces of the furnace are inhibited against the formation of a catalytically active coke during the processing of the hydrocarbon feedstock. A thin catalytically inactive coke layer is also formed on the surfaces in contact with the hydrocarbon feedstock downstream of the radiant heating section of the pyrolysis furnace.

Some examples of the inhibiting compound include, but are not limited to, ethylbenzene, n-propylbenzene, i-propylbenzene, n-butylbenzene, and t-butylbenzene.

The addition of inhibiting compound to the pyrolysis furnace can be started prior to the processing of a hydrocarbon feedstock, and may be continued during the processing of a hydrocarbon feedstock, or may be started during the processing of the hydrocarbon feedstock. The inhibiting compound may be added to the furnace on a continuous or intermittent basis prior to or during the processing of a hydrocarbon feedstock. The addition of the inhibiting compound may be discontinued prior to or during the processing of the hydrocarbon feedstock.

The inhibiting compound may be further defined as having the following formulae:

I.

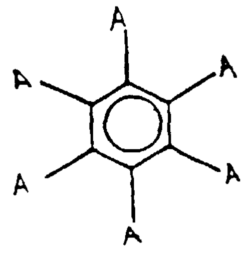

II.

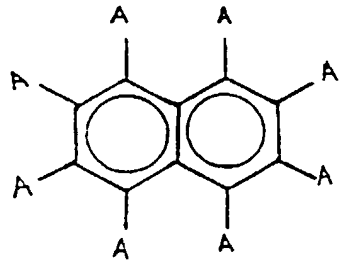

III.

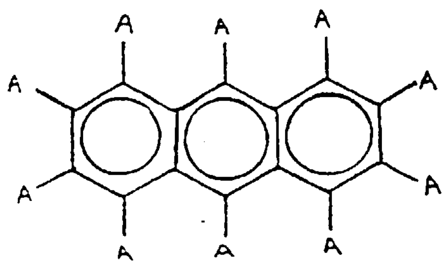

IV.

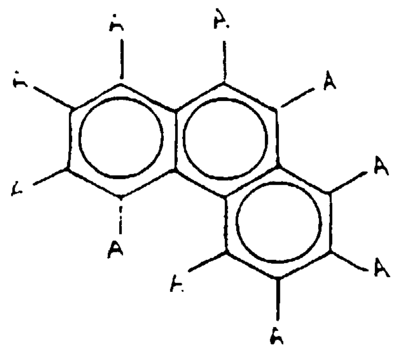

A is selected from the group consisting of hydrogen and Z wherein at least one occurrence of A must be Z. Z is a substituent having the formula

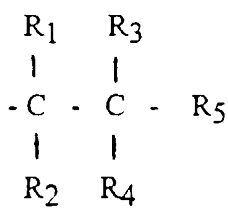

wherein $R_1$, $R_2$, $R_3$, $R_4$, and $R_5$ may be the same as or different from each other and are independently selected from the group consisting of hydrogen, alkyl, alkene, cycloalkyl, alkyne, alkylaryl, aryl, and arylalkyl, $R_1$, $R_2$, $R_3$, $R_4$, and $R_5$ each may contain up to 15 carbon atoms.

Alternatively, Z is a substituent having the formula:

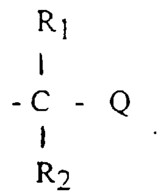

wherein Q is selected from the group consisting of: O-$R_3$, hydrogen, alkyl, alkene, cycloalkyl, alkyne, alkylaryl, aryl, arylalkyl, $R_1$, $R_2$, $R_3$, and $R_4$ may be the same as or different from each other and are independently selected from the group consisting of hydrogen, alkyl, alkene, cycloalkyl, alkyne, alkylaryl, aryl, arylalkyl, $R_1$, $R_2$, $R_3$, $R_4$, and Q may each contain up to 15 carbon atoms. However, $R_1$, $R_2$, and Q are not each a hydrogen atom at the same time.

The inhibiting compound may be formulated in an organic solvent or an aqueous solution. The inhibiting compound may be added to the furnace in a carrier selected from the group consisting of steam, hydrocarbon gases, inert gases, and mixtures thereof. The term carrier as used herein, includes the fluids or gases that are typically present in the furnace environment as well as fluids or gases that may be added specifically to carry the inhibiting compound into the furnace. During the addition of the inhibiting compound to the pyrolysis furnace, the furnace is maintained at a temperature ranging from about 500 to about 1200°C, and more preferably, at a temperature ranging from about 700 to about 1100°C. The hydrocarbon feedstock includes at least one fraction selected from the group consisting of ethane, propane, butane, naphtha, kerosene, and gas oil.

In another embodiment of the invention, the method comprises processing a hydrocarbon feedstock in the presence of an inhibiting compound selected from the group consisting of substituted benzenes, substituted naphthalenes, substituted anthracenes, substituted phenanthrenes, and mixtures thereof. The inhibiting compound contains at least one hydrocarbon substituent having at least 2 carbon atoms.

A thin catalytically inactive coke layer is formed on the surfaces of the pyrolysis furnace, whereby the surfaces of the furnace are inhibited against formation of a catalytically active coke during the processing of a hydrocarbon feedstock. A thin catalytically inactive coke layer is also formed on the surfaces in contact with the hydrocarbon feedstock downstream of the radiant heating section of the pyrolysis furnace.

The run length of the pyrolysis furnace used to process hydrocarbon feedstock may be increased by decoking the pyrolysis furnace, and prior to processing the hydrocarbon feedstock, adding a said inhibiting compound to the furnace. A thin catalytically inactive coke layer is formed on the surfaces of the pyrolysis furnace having contact with the hydrocarbon feedstock. The hydrocarbon feedstock is then fed into the furnace. The surfaces of the furnace are inhibited against the formation of catalytically active coke during the processing of the hydrocarbon feedstock thereby increasing the run length of the pyrolysis furnace.

The product yield from the processing of a hydrocarbon feedstock through a pyrolysis furnace may be increase by decoking the pyrolysis furnace, and prior to processing a hydrocarbon feedstock, adding to the pyrolysis furnace a said inhibiting compound. A thin catalytically inactive coke layer is formed on the surfaces of the pyrolysis furnace having contact with the hydrocarbon feedstock. The hydrocarbon feedstock is then fed into the furnace. The surfaces of the furnace are inhibited against the formation of catalytically active coke during the processing of the hydrocarbon feedstock thereby increasing the product yield from the processing of the hydrocarbon feedstock through the pyrolysis furnace.

The present invention discloses a method of using aromatic-containing inhibiting compounds and a discovery of the most effective formula of aromatics to reduce the coke deposition in pyrolysis furnaces in which hydrocarbon feedstocks are thermally converted to ethylene as well as other useful products at temperatures ranging from 700 to 1100° C. By combining this method and an effective formula, a significant reduction in coke deposition is achieved.

In this method, the additive treatment starts during a stand-by which is the time between furnace decoking and the introduction of hydrocarbon feedstock. Conventionally, during stand-by, steam is continuously being added to the furnace. A sufficient amount of the aromatic-based coke inhibiting compound is brought in contact with the radiant coil reactor surface of a pyrolysis furnace for a certain time prior to processing a hydrocarbon feedstock (pretreatment). During the pretreatment, the operation conditions are generally mild relative to those under which hydrocarbon feedstocks are processed, i.e., lower temperature (stand-by temperature) and steam dominated environment (because no hydrocarbon feedstock is present). This mild operation condition is very important for the development of a low defect, stable and effective catalytically inactive coke layer.

The addition of the coke inhibiting compound may be continued through start-up of the processing of the hydrocarbon feedstock. Preferably, the addition is terminated at a certain point where a steady cracking operation condition is reached, even though continuous or intermittent addition is also acceptable.

The aromatic inhibiting compounds are used to treat the inner surface of the radiant section reactor tubes of

the pyrolysis furnace and inner surfaces of sections downstream of the radiant section reactor which are in contact with the hydrocarbon feedstock. By adding the inhibiting compound, a thin catalytically inactive coke layer is formed on the surfaces of the pyrolysis furnace. The thickness of the coke layer can range from about a molecular thickness to a level of which does not substantially restrict the flow of the hydrocarbon feedstock through the pyrolysis furnace. The catalytically inactive coke layer prevents coke precursors from contacting the surface of the pyrolysis furnace during the processing of a hydrocarbon feedstock, and thus, inhibits the formation of catalytically active coke, whereby the coke formation and deposition on the surfaces of the furnace is reduced during processing of the hydrocarbon feedstock. A thin catalytically inactive coke layer is also formed on the surfaces in contact with the hydrocarbon feedstock downstream of the radiant heating section of the pyrolysis furnace.

Addition of the inhibiting compound is usually started prior to the processing of a hydrocarbon feedstock. The addition of the inhibiting compound may be discontinued prior to or during the processing of the hydrocarbon feedstock. It is preferred that the addition of the inhibiting compound is terminated once a steady operating condition is established during the processing of the hydrocarbon feedstock. The addition of the inhibiting compound may also be started during the processing of the hydrocarbon feedstock. The inhibiting compound may be added on a continuous or intermittent basis before and/or during the processing of a hydrocarbon feedstock.

The possible point of injection of the inhibiting compound is unimportant as long as fouling due to the presence of the inhibiting compound is not a concern. Where fouling due to the presence of the inhibiting compound is a concern, such as in the convection section of the furnace, the inhibiting compound is preferably added to the furnace from anywhere after the place where hydrocarbon and dilution steam are mixed together but before the inlet to the radiant section. In general, it is most preferred to install the injection nozzles as close to the radiant section as possible. The objective of selecting injection locations is to ensure that no adverse effect, such as fouling in the early convection section such as that caused by insufficient vaporization, will occur from the use of the inhibiting compounds.

The surfaces can be treated with the inhibiting compound in several different ways, including for example, pretreating the surfaces prior to admitting hydrocarbon feedstocks (pretreatment), or continuously or intermittently adding the inhibiting compound to the hydrocarbon feedstock as it is being processed (continuous or intermittent treatment). A combination of the pretreatment with either the continuous or the intermittent treatment is preferred.

A pretreatment is conducted during the stand-by after decoking and prior to admitting hydrocarbon feedstocks. During a pretreatment, the inhibiting compound is carried into the furnace by a carrier. Preferred dosage ranges from 100 parts per million (ppm) up to 50% on the basis of the carrier mass flow, more preferably from 1000 ppm to 20%.

During a continuous or intermittent treatment, the aromatics are preferably added at a rate from about 100 ppm to 10% on the basis of the hydrocarbon feed mass flow, more preferably from about 1000 ppm to 1%. The dosage and the duration of the inhibiting compound treatment have to be carefully chosen and controlled. Excess use or extraordinary long addition of the inhibiting compound may result in too much coke deposition or fouling in radiant coils and/or TLEs.

The preferred inhibiting compounds are the molecules containing alkyl benzenes, alkyl naphthalenes, and alkyl triaromatics (such as anthracenes or phenanthrenes). The alkyl substituents may contain double or triple bonds, and/or cycloalkyls as well as aryl groups. The inhibiting compounds may also contain more than one substituent per benzene ring (i.e., di, tri, or tetra substituted, etc). Using the treatment method disclosed above, the present invention found that most hydrocarbons, aromatics or paraffins, are effective to different extents in coke inhibition. However, the aromatic inhibiting compounds are generally more efficient than paraffinics.

The most important discovery of the present invention is that the most efficient coke inhibiting compounds of aromatics are those substituted aromatics from which benzyl-based radicals, $Ph\text{-}CR_1R_2\bullet$, are easily formed under mild thermal conditions. $R_1$ and $R_2$ are H and/or alkyl groups. The alkyl substituent may contain unsaturated and cycloalkyl moieties anywhere along the substituent.

Even though alkyl naphthalenes and alkyl substituted polyaromatics are the preferred aromatics, care should be taken concerning their high melting and boiling points and their higher fouling tendency. The present invention recognized that coke inhibition efficiency could be further improved by blending alkyl aromatics of different structures. It is believed that the use of such aromatics will generate a well-packed, low defect coke layer which effectively isolates gas phase coke precursors from active surface sites.

The advantage of the aromatics-based coke inhibiting compounds over other type coke inhibitors is obvious to those familiar with the hydrocarbon pyrolysis processes. Most of the coke inhibitors in the current literature are based on applying certain chemicals or elements which are not typically present in the process stream. The introduction of such coke inhibitors into the process often raises concerns about their effect on reactor tube metallurgy, their interference with cracking reaction kinetics, their potential contamination on downstream processes, and their removal from the process. On the other hand, the use of the present aromatic-based coke inhibiting compounds does not have any of the concerns mentioned above because they do not introduce any foreign elements into the process, and aromatics are part of the cracked products.

The first contribution of this invention is the discovery of the most effective aromatics with respect to coking

reduction. The effectiveness means that these inhibiting compounds can develop an effective catalytically inactive coke layer within a reasonable time.

The second contribution of this invention is the selection of the injection point. Aromatics are known fouling precursors, especially in an environment where only a small amount or no steam is present. This is often the situation in a convection section for most of the current pyrolysis furnaces. If the inhibiting compounds are improperly added before this section, fouling could occur in this section due to insufficient vaporization, which would adversely affect the operation of pyrolysis furnaces.

The third contribution of this invention is the treatment method and procedure. The invention recognized the importance of pretreatment and the additional benefits of a combination of pretreatment with either continuous or intermittent addition when using the inhibiting compounds as coke reduction additives. The invention also identified the proper conditions for pretreatment. The method ensures that the surface will be well passivated before contacting hydrocarbon feedstocks, and furthermore, the passivation will be preserved thereafter during the cracking operation.

The method also realizes the importance of terminating the addition of the inhibiting compound as soon as an effective catalytically inactive coke layer is established on the surfaces of the pyrolysis furnace. This is because extensive use of this inhibiting compound, either by high dosage or long addition duration, will make the coke layer too thick, which will raise concerns about coke spalling and plugging.

The following examples are presented to describe preferred embodiments and utilities of the invention and are not meant to limit the invention unless otherwise stated.

The test method involved the utilization of a bench-scale laboratory cracking reaction unit which simulated the operations in a pyrolysis furnace. The furnace reactor of this simulation unit consisted of a stainless steel coil preheater (convection section), a quartz tube reactor (radiant section) and an electrobalance. A test coupon of Incoloy 800 alloy was suspended in the radiant section of the furnace reactor, and its weight was constantly recorded by the electrobalance. The weight increase during a cracking operation was an indication of coke deposition on the metal coupon. The typical output from the electrobalance was a plot of coke buildup vs. time on stream. A coking rate vs. time plot was obtained by differentiating the coke buildup vs. time plot.

Steam was always present in the process stream of the furnace reactor during hot stand-by, cracking run and decoking operations. A cracking run was initiated by introducing a hydrocarbon feedstock at a stand-by temperature (ca. 800° C). The radiant reactor temperature was then increased from the stand-by temperature to a cracking run temperature (from about 920 to about 940° C) and then maintained at that temperature. The hydrocarbon feedstock was ethane with 40 ppm $H_2S$. The steam to hydrocarbon weight ratio was 0.30-0.35. The residence time was about 0.3 second for cracking run. A decoking operation was performed in an air-steam environment at about 800 to about 810° C.

A coke inhibitor additive was applied by introducing the additive at the front of the radiant reactor. The injection of the additive, the inhibiting compound, started within a certain time prior to admitting hydrocarbon feed. The addition was terminated after the cracking run reached a steady state.

Blank Experiments

A blank run, in which no coke inhibitor treatment was applied, is shown in Figure 1 in terms of coke buildup vs. time, and the corresponding coking rate vs. time plot is given in the same figure. The initial fast increase in coking rate was due to the temperature ramp from the stand-by (800° C) to the cracking reaction (940° C) temperatures. After the coking rate reached its maximum at the end of the temperature ramp, a fast decline in coking rate was observed, which is consistent with conventional coking kinetics on an active metal surface. Generally, the change in coking rate was insignificant after two hours on stream, i.e., coking rate reached a steady state, asymptotic coking rate.

Additive Treated Runs

Under the same experimental conditions as the blank run shown above, several additives were evaluated regarding their efficiency on coke deposition reduction.

These additives were applied in the way described above. The additive treatments were continued through the start-up, and they were terminated after a steady cracking operation condition had been reached. Coking rates in Table 1 were recorded when a steady coking rate was reached. The percentage of coking rate reduction in Table 1 is defined as:

$$(1 - \text{ratio of coking rates of a treated run to a blank run}) \times 100\%$$

Additive A was a mixture of o-, m- and p-xylenes. Additive B was a mixture of aromatics cycloalkanes and

paraffins with a total aromatic content of 65%. Alkyl benzenes and alkyl naphthalenes were the major components in Additive B.

As Table I indicates, all the hydrocarbon additives showed inhibition of coke formation through this type treatment, and aromatics, in general, were more effective. Of the aromatics, the performance was significantly improved from toluene to t-butylbenzene. It was noticed that Additive A behaved quite similar to toluene, and it was also found that there was no notable difference between n-butylbenzene and t-butylbenzene concerning coke inhibition. The similarity between toluene and xylenes and between n-butylbenzene and t-butylbenzene and the difference between the methyl and the butyl substituted benzenes suggests that the performance depends on the ease of forming a benzyl radical, i.e., thermally, it is easier to generate a benzyl radical from butylbenzene than toluene or xylene. As Table I shows, the performance was further enhanced by using a mixture of alkyl benzenes and alkyl naphthalenes (Additive B).

It was also found that not all aromatics provided the same benefit, and some of them even showed adverse effect in coke inhibition. Naphthalene and styrene were such examples, and a significant increase in coking rate was observed when either one of them was used as a treatment additive.

The influence of Additive B on overall coking rate is presented in Figure 2, compared to the blank run. It is obvious that the Additive B treatment not only lowered the asymptotic coking rate (Table I), but also totally eliminated the initial fast coke build up regime.

TABLE I

| REDUCTION IN COKING RATE | |
|---|---|
| ADDITIVES | COKING RATE REDUCTION,% |
| blank | 0 |
| n-pentane | 35 |
| n-dodecane | 35 |
| toluene | 43 |
| Additive A | 53 |
| t-butylbenzene | 85 |
| Additive B | 91 |

## Claims

1. A method for inhibiting the formation of coke on the surfaces of a radiant heating section of a pyrolysis furnace and the surfaces immediately downstream of such section in contact with hydrocarbon feedstock which comprises:

   a. decoking the pyrolysis furnace;
   b. prior to processing a hydrocarbon feedstock, adding to the pyrolysis furnace a hydrocarbon inhibiting compound selected from the group consisting of:

      1. substituted benzenes;
      2. substituted naphthalenes;
      3. substituted anthracenes;
      4. substituted phenanthrenes; and,
      5. mixtures thereof,

   wherein the inhibiting compound contains at least one hydrocarbon substituent having at least 2 carbon atoms;
   c. forming a thin catalytically inactive coke layer on the surfaces of the pyrolysis furnace and the surfaces immediately downstream of the furnace; and then,
   d. feeding the hydrocarbon feedstock to the furnace,

whereby the surfaces of said furnace are inhibited against formation of a catalytically active coke during the processing of a hydrocarbon feedstock.

**2.** The method according to Claim 1, wherein the addition of the inhibiting compound is discontinued during processing the hydrocarbon feedstock.

**3.** The method according to Claim 1, wherein the addition of the inhibiting compound is discontinued prior to processing the hydrocarbon feedstock.

**4.** The method according to Claim 1, wherein the inhibiting compound is added intermittently prior to the processing of the hydrocarbon feedstock.

**5.** The method according to Claim 1, wherein the inhibiting compound is added continuously prior to the processing of the hydrocarbon feedstock.

**6.** The method according to Claim 1, wherein the inhibiting compound is added to the furnace in a carrier selected from the group consisting of:

a. steam;
b. hydrocarbon gases;
c. inert gases; and,
d. mixtures thereof.

**7.** The method according to Claim 1, wherein the inhibiting compound is added in a range of from 100 ppm to 50% on the basis of carrier mass flow prior to processing hydrocarbon feedstock.

**8.** The method according to Claim 1, wherein during the addition of the inhibiting compound, the furnace is maintained at a temperature ranging from 700 to 1100°C.

**9.** A method for inhibiting the formation of coke on the surfaces of a radiant heating section of a pyrolysis furnace and the surfaces immediately downstream of such section in contact with hydrocarbon feedstock which comprises:

a. processing of a hydrocarbon feedstock in the presence of a hydrocarbon inhibiting compound selected from the group consisting of:

1. substituted benzenes;
2. substituted naphthalenes;
3. substituted anthracenes;
4. substituted phenanthrenes; and,
5. mixtures thereof,

wherein the inhibiting compound contains at least one hydrocarbon substituent having at least 2 carbon atoms; and,
b forming a catalytically inactive thin coke layer on the surfaces of the pyrolysis furnace,

whereby the surfaces of the furnace are inhibited against formation of a catalytically active coke during the processing of a hydrocarbon feedstock.

**10.** A method according to any of claims 1 to 9 wherein the inhibiting compound is selected from the group having the following formulae:

I.

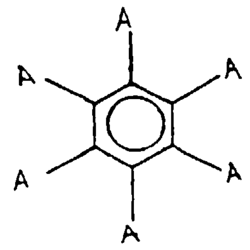

II.

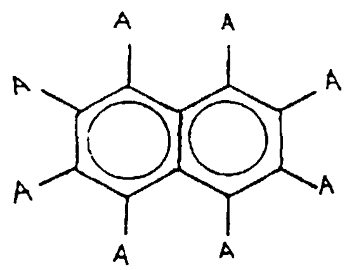

III.

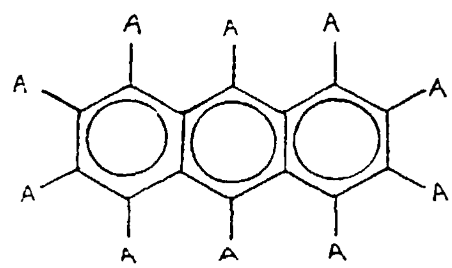

IV.

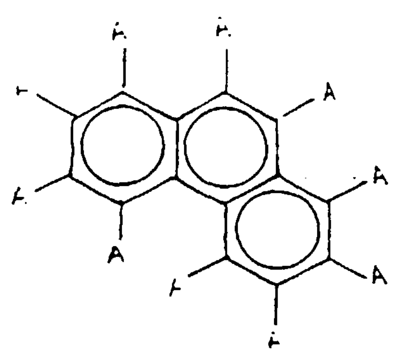

wherein A is selected from the group consisting of: hydrogen and Z and at least one occurrence of A must be Z wherein Z is a substituent having the formula:

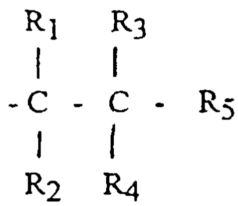

wherein $R_1$, $R_2$, $R_3$, $R_4$, and $R_5$ may be the same as or different from each other and are independently selected from the group consisting of: hydrogen, alkyl, alkene, cycloalkyl, alkyne, alkylaryl, aryl and arylalkyl, wherein $R_1$, $R_2$, $R_3$, $R_4$, and $R_5$ each contain up to 15 carbon atoms.

**11.** A method according to any of claims 1 to 9 wherein the inhibiting compound is selected from the group having the following formulae:

I.

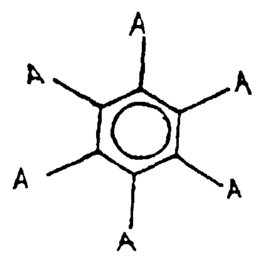

II.

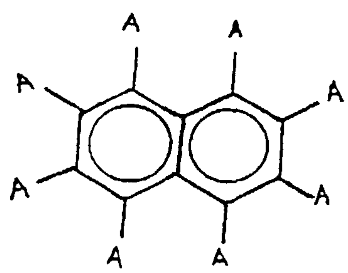

III.

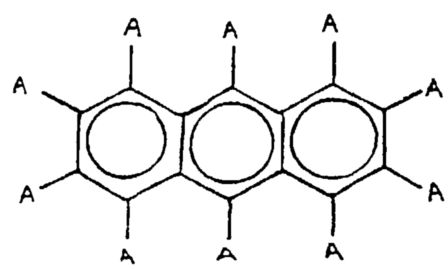

IV.

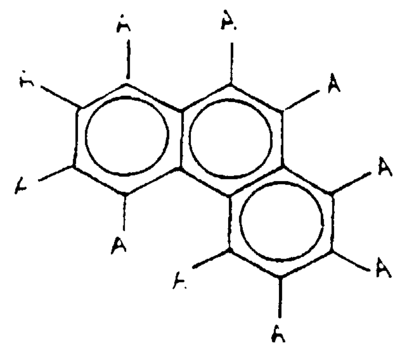

wherein A is selected from the group consisting of: hydrogen and Z and at least one occurrence of A must be Z wherein Z is a substituent having the formula:

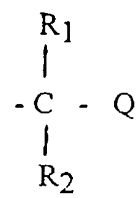

wherein Q is selected from the group consisting of: hydrogen, alkyl, alkene, cycloalkyl, alkyne, alkylaryl, aryl and arylalkyl, and wherein $R_1$, $R_2$, $R_3$, and $R_4$ may be the same as or different from each other and are independently selected from the group consisting of: hydrogen, alkyl, alkene, cycloalkyl, alkyne, alkylaryl, aryl, arylalkyl, and wherein $R_1$, $R_2$, $R_3$, $R_4$, and Q each contain up to 15 carbon atoms and $R_1$, $R_2$, and Q are not all a hydrogen atom at the same time.

**12.** The method according to any of claims 1 to 11 wherein the inhibiting compound is in an organic solvent.

**13.** The method according to any of claims 1 to 11 wherein the inhibiting compound is in an aqueous solution.

**14.** The method according to claim 1 or claim 9 wherein the inhibiting compound is added intermittently during the processing of the hydrocarbon feedstock.

**15.** The method according to claim 1 or claim 9 wherein the inhibiting compound is added continuously during the processing of the hydrocarbon feedstock.

**16.** The method according to any of claims 1 to 15 wherein the hydrocarbon feedstock contains at least one fraction selected from the group consisting of:

a. ethane;
b. propane;
c. butane;
d. naphtha;
e. kerosene; and
f. gas oil.

**17.** The method according to any of claims 1 to 16 wherein a thin catalytically inactive coke layer is formed on the surfaces in contact with the hydrocarbon feedstock downstream of the radiant heating section.

**18.** The method according to any of claims 1 to 17 wherein the inhibiting compound is added in a range of from 100 ppm to 10% on the basis of hydrocarbon feedstock mass flow during the processing of the hydrocarbon feedstock.

**19.** The method according to any of claims 1 to 18 wherein during the addition of the inhibiting compound, the furnace

is maintained at a temperature ranging from 500 to 1200°C.

## Patentansprüche

1. Verfahren zum Hemmen der Koksbildung an den Oberflächen eines Strahlungsheizabschnitts eines Pyrolyseofens und den Oberflächen unmittelbar stromab von einem solchen Abschnitt, die mit Kohlenwasserstoff-Feedmaterial in Kontakt stehen, wobei das Verfahren umfasst:

    a) das Entkoken des Pyrolyseofens;
    b) die Zufuhr einer Kohlenwasserstoff-Inhibitorverbindung, ausgewählt aus der aus:

        1. substituierten Benzolen;
        2. substituierten Naphthalinen;
        3. substituierten Anthracenen;
        4. substituierten Phenanthrenen; und
        5. Gemischen davon,

    bestehenden Gruppe, worin die Inhibitorverbindung zumindest einen Kohlenwasserstoffsubstituenten mit zumindest 2 Kohlenstoffatomen enthält, zum Pyrolyseofen vor dem Verarbeiten eines Kohlenwasserstoff-Feedmaterials;
    c) das Ausbilden einer dünnen, katalytisch inaktiven Koksschicht auf den Oberflächen des Pyrolyseofens und den Oberflächen unmittelbar stromab vom Ofen; und dann
    d) die Zufuhr des Kohlenwasserstoff-Feedmaterials zum Ofen,

    wodurch die Bildung von katalytisch aktivem Koks an den Oberflächen des Ofens während des Verarbeitens eines Kohlenwasserstoff-Feedmaterials gehemmt wird.

2. Verfahren nach Anspruch 1, worin die Zufuhr der Inhibitorverbindung während der Verarbeitung des Kohlenwasserstoff-Feedmaterials unterbrochen wird.

3. Verfahren nach Anspruch 1, worin die Zufuhr der Inhibitorverbindung vor der Verarbeitung des Kohlenwasserstoff-Feedmaterials unterbrochen wird.

4. Verfahren nach Anspruch 1, worin die Inhibitorverbindung vor der Verarbeitung des Kohlenwasserstoff-Feedmaterials intermittierend zugeführt wird.

5. Verfahren nach Anspruch 1, worin die Inhibitorverbindung vor der Verarbeitung des Kohlenwasserstoff-Feedmaterials kontinuierlich zugeführt wird.

6. Verfahren nach Anspruch 1, worin die Inhibitorverbindung dem Ofen in einem Träger zugegeben wird, der aus der aus:

    a) Dampf;
    b) Kohlenwasserstoffgasen;
    c) Inertgasen; und
    d) Gemischen davon

    bestehenden Gruppe ausgewählt ist.

7. Verfahren nach Anspruch 1, worin die Inhibitorverbindung in einem Bereich von 100 ppm bis 50 %, bezogen auf den Trägermassenstrom, vor der Verarbeitung von Kohlenwasserstoff-Feedmaterial zugeführt wird.

8. Verfahren nach Anspruch 1, worin während der Zufuhr der Inhibitorverbindung der Ofen auf einer Temperatur im Bereich von 700 bis 1.100 °C gehalten wird.

9. Verfahren zum Hemmen der Koksbildung an den Oberflächen eines Strahlungsheizabschnitts eines Pyrolyseofens und an den Oberflächen unmittelbar stromab von einem solchen Abschnitt, die mit Kohlenwasserstoff-Feedmaterial in Kontakt stehen, wobei das Verfahren umfasst:

a) das Verarbeiten eines Kohlenwasserstoff-Feedmaterials in Gegenwart einer Kohlenwasserstoff-Inhibitorverbindung, die aus der aus:

1) substituierten Benzolen;
2) substituierten Naphthalinen;
3) substituierten Anthracenen;
4) substituierten Phenanthrenen; und
5) Gemischen davon

bestehenden Gruppe ausgewählt ist, worin die Inhibitorverbindung zumindest einen Kohlenwasserstoff-Substituenten enthält, der zumindest 2 Kohlenstoffatome aufweist; und
b) das Ausbilden einer katalytisch inaktiven, dünnen Koksschicht auf den Oberflächen des Pyrolyseofens,

wodurch die Bildung von katalytisch aktivem Koks an den Oberflächen des Ofens während der Verarbeitung eines Kohlenwasserstoff-Feedmaterials gehemmt wird.

**10.** Verfahren nach einem der Ansprüche 1 bis 9, worin die Inhibitorverbindung aus der Gruppe mit den folgenden Formeln ausgewählt ist:

I.

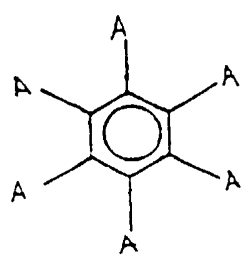

II.

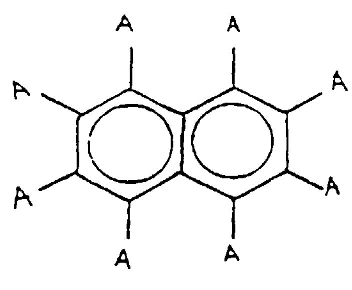

III.

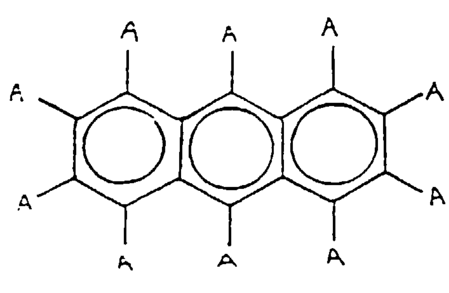

IV.

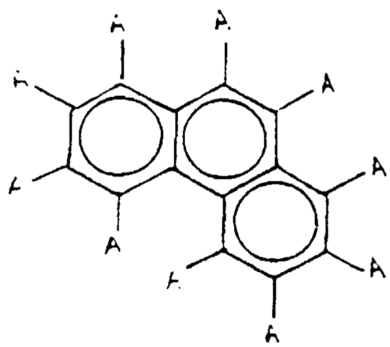

worin A aus der aus Wasserstoff und Z bestehenden Gruppe ausgewählt ist und zumindest in einem Fall A = Z sein muss, worin Z ein Substituent der Formel:

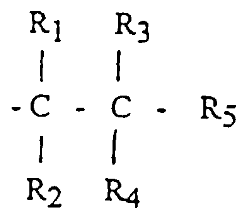

ist, worin $R_1$, $R_2$, $R_3$, $R_4$ und $R_5$ gleich oder voneinander verschieden sein können und unabhängig voneinander aus der aus Wasserstoff, Alkyl, Alken, Cycloalkyl, Alkin, Alkylaryl, Aryl und Arylalkyl bestehenden Gruppe ausgewählt sind, worin $R_1$, $R_2$, $R_3$, $R_4$ und $R_5$ jeweils bis zu 15 Kohlenstoffatome enthalten.

**11.** Verfahren nach einem der Ansprüche 1 bis 9, worin die Inhibitorverbindung aus der Gruppe mit den folgenden Formeln ausgewählt ist:

I.

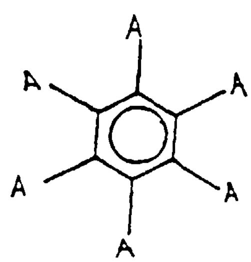

II.

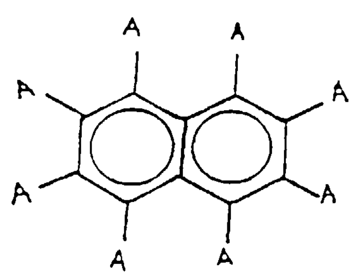

III.

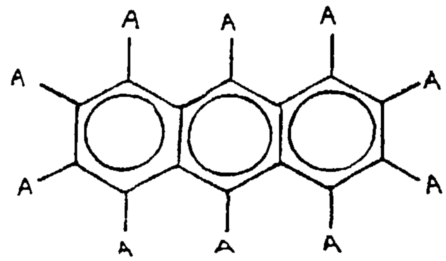

IV.

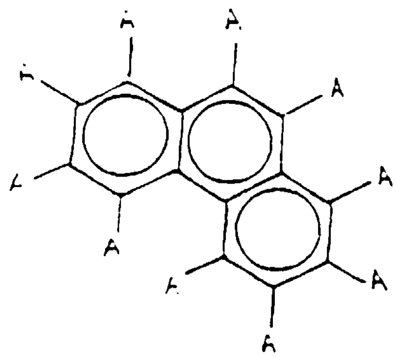

worin A aus der aus Wasserstoff und Z bestehenden Gruppe ausgewählt ist und zumindest in einem Fall A = Z sein muss, worin Z ein Substituent der Formel:

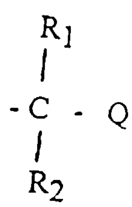

ist, worin Q aus der aus Wasserstoff, Alkyl, Alken, Cycloalkyl, Alkin, Alkylaryl, Aryl und Arylalkyl bestehenden Gruppe ausgewählt ist, worin $R_1$, $R_2$, $R_3$ und $R_4$ gleich oder voneinander verschieden sein können und unabhängig voneinander aus der aus Wasserstoff, Alkyl, Alken, Cycloalkyl, Alkin, Alkylaryl, Aryl und Arylalkyl bestehenden Gruppe ausgewählt sind, und worin $R_1$, $R_2$, $R_3$, $R_4$ und Q jeweils bis zu 15 Kohlenstoffatome enthalten und $R_1$, $R_2$ und Q nicht alle gleichzeitig Wasserstoffatome sind.

**12.** Verfahren nach einem der Ansprüche 1 bis 11, worin die Inhibitorverbindung in einem organischen Lösungsmittel vorliegt.

**13.** Verfahren nach einem der Ansprüche 1 bis 11, worin die Inhibitorverbindung in einer wässrigen Lösung vorliegt.

**14.** Verfahren nach Anspruch 1 oder Anspruch 9, worin die Inhibitorverbindung während der Verarbeitung des Kohlenwasserstoff-Feedmaterials intermittierend zugeführt wird.

**15.** Verfahren nach Anspruch 1 oder Anspruch 9, worin die Inhibitorverbindung während der Verarbeitung des Kohlenwasserstoff-Feedmaterials kontinuierlich zugeführt wird.

**16.** Verfahren nach einem der Ansprüche 1 bis 15, worin das Kohlenwasserstoff-Feedmaterial zumindest eine Fraktion enthält, die aus der aus:

a) Ethan;

b) Propan;
c) Butan;
d) Naphtha;
e) Kerosin; und
f) Dieselöl

bestehenden Gruppe ausgewählt ist.

**17.** Verfahren nach einem der Ansprüche 1 bis 16, worin eine dünne, katalytisch inaktive Koksschicht an den Oberflächen stromab vom Strahlungsheizabschnitt ausgebildet wird, die mit dem Kohlenwasserstoff-Feedmaterial in Kontakt stehen.

**18.** Verfahren nach einem der Ansprüche 1 bis 17, worin die Inhibitorverbindung während der Verarbeitung des Kohlenwasserstoff-Feedmaterials in einem Bereich von 100 ppm bis 10 %, bezogen auf den Kohlenwasserstoff-Feedmaterial-Massenstrom, zugeführt wird.

**19.** Verfahren nach einem der Ansprüche 1 bis 18, worin während der Zufuhr der Inhibitorverbindung der Ofen auf einer Temperatur im Bereich von 500 bis 1.200 °C gehalten wird.

**Revendications**

**1.** Procédé pour inhiber la formation de coke sur les surfaces d'une section de chauffage à rayonnement d'un four de pyrolyse et sur les surfaces directement en aval de ladite section entrant en contact avec la charge d'alimentation en hydrocarbure, qui comprend le fait de:

a. soumettre le four de pyrolyse à un décokage;
b. avant de traiter une charge d'alimentation en hydrocarbure, ajouter au four de pyrolyse un composé d'inhibition hydrocarburé choisi parmi le groupe constitué par:

1. des benzènes substitués;
2. des naphtalènes substitués;
3. des anthracènes substitués;
4. des phénanthrènes substitués; et
5. leurs mélanges,

le composé d'inhibition contenant au moins un substituant d'hydrocarbure possédant au moins deux atomes de carbone;
c. former une mince couche de coke dépourvue d'activité catalytique sur les surfaces du four de pyrolyse et sur les surfaces immédiatement en aval du four; puis
d. introduire la charge d'alimentation en hydrocarbure dans le four,

par lequel on inhibe la formation de coke doté d'une activité catalytique sur les surfaces dudit four au cours du traitement d'une charge d'alimentation en hydrocarbure.

**2.** Procédé selon la revendication 1, dans lequel l'addition du composé d'inhibition est interrompue au cours du traitement de la charge d'alimentation en hydrocarbure.

**3.** Procédé selon la revendication 1, dans lequel l'addition du composé d'inhibition est interrompue avant le traitement de la charge d'alimentation en hydrocarbure.

**4.** Procédé selon la revendication 1, dans lequel le composé d'inhibition est ajouté par intermittence avant le traitement de la charge d'alimentation en hydrocarbure.

**5.** Procédé selon la revendication 1, dans lequel le composé d'inhibition est ajouté en continu avant le traitement de la charge d'alimentation en hydrocarbure.

**6.** Procédé selon la revendication 1, dans lequel le composé d'inhibition est ajouté au four dans un support choisi

parmi le groupe constitué par:

a. de la vapeur;
b. des gaz d'hydrocarbures;
c. des gaz inertes; et
d. leurs mélanges.

**7.** Procédé selon la revendication 1, dans lequel le composé d'inhibition est ajouté dans le domaine de 100 ppm à 50% sur base du débit massique du support avant de traiter la charge d'alimentation en hydrocarbure.

**8.** Procédé selon la revendication 1, dans lequel, au cours de l'addition du composé d'inhibition, on maintient le four à une température se situant dans le domaine de 700 à 1100°C.

**9.** Procédé pour inhiber la formation de coke sur les surfaces d'une section de chauffage à rayonnement d'un four de pyrolyse et sur les surfaces directement en aval de ladite section entrant en contact avec la charge d'alimentation en hydrocarbure, qui comprend le fait de:

a. traiter une charge d'alimentation en hydrocarbure en présence d'un composé d'inhibition hydrocarburé choisi parmi le groupe constitué par:

1. des benzènes substitués;
2. des naphtalènes substitués;
3. des anthracènes substitués;
4. des phénanthrènes substitués; et
5. leurs mélanges,

le composé d'inhibition contenant au moins un substituant d'hydrocarbure possédant au moins deux atomes de carbone;
b. former une mince couche de coke dépourvue d'activité catalytique sur les surfaces du four de pyrolyse,

par lequel on inhibe la formation de coke doté d'une activité catalytique sur les surfaces du four au cours du traitement d'une charge d'alimentation en hydrocarbure.

**10.** Procédé selon l'une quelconque des revendications 1 à 9, dans lequel le composé d'inhibition est choisi parmi le groupe répondant aux formules ci-après:

I.

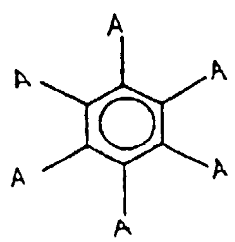

II.

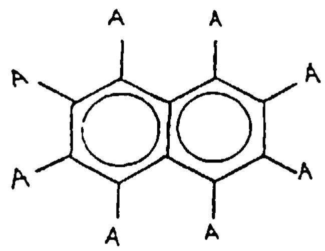

III.

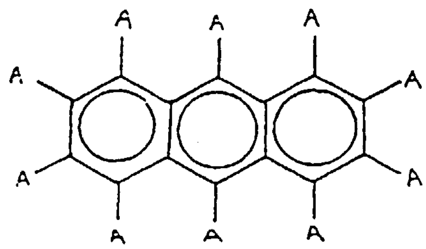

IV.

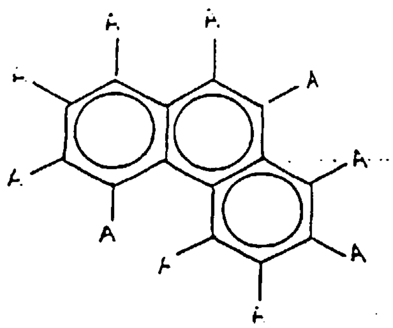

dans lesquelles A est choisi parmi le groupe constitué par un atome d'hydrogène et par Z, A devant représenter au moins une fois Z et Z représentant un substituant répondant à la formule:

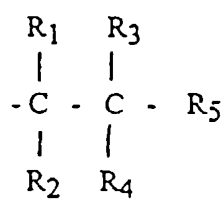

dans laquelle $R_1$, $R_2$, $R_3$, $R_4$ et $R_5$ peuvent être identiques ou différents l'un de l'autre et sont choisis, indépendamment l'un de l'autre, parmi le groupe constitué par un atome d'hydrogène, un groupe alkyle, un groupe alcène, un groupe cycloalkyle, un groupe alcyne, un groupe alkylaryle, un groupe aryle et un groupe arylalkyle, $R_1$, $R_2$, $R_3$, $R_4$ et $R_5$ contenant chacun jusqu'à 15 atomes de carbone.

**11.** Procédé selon l'une quelconque des revendications 1 à 9, dans lequel le composé d'inhibition est choisi parmi le groupe répondant aux formules ci-après:

I.

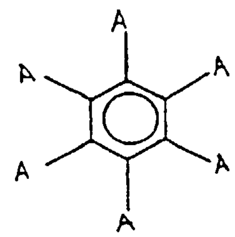

II.

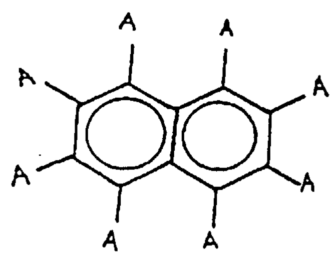

III.

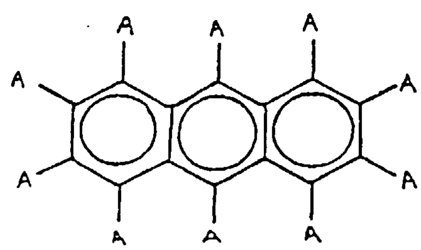

IV.

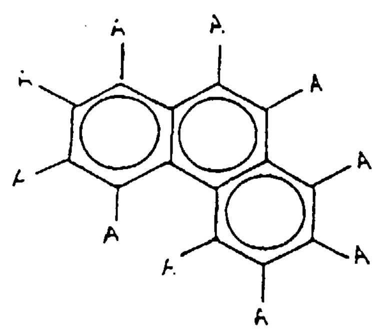

dans lesquelles A est choisi parmi le groupe constitué par un atome d'hydrogène et par Z, A devant représenter au moins une fois Z et Z représentant un substituant répondant à la formule:

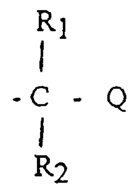

dans laquelle Q est choisi parmi le groupe constitué par un atome d'hydrogène, un groupe alkyle, un groupe alcène, un groupe cycloalkyle, un groupe alcyne, un groupe alkylaryle, un groupe aryle et un groupe arylalkyle, et $R_1$, $R_2$, $R_3$, $R_4$ peuvent être identiques ou différents l'un de l'autre et sont choisis, indépendamment l'un de l'autre, parmi le groupe constitué par un atome d'hydrogène, un groupe alkyle, un groupe alcène, un groupe cycloalkyle, un groupe alcyne, un groupe alkylaryle, un groupe aryle, un groupe arylalkyle, et $R_1$, $R_2$, $R_3$, $R_4$ et Q contiennent chacun jusqu'à 15 atomes de carbone, $R_1$, $R_2$ et Q ne représentant pas un atome d'hydrogène tous en même temps.

**12.** Procédé selon l'une quelconque des revendications 1 à 11, dans lequel le composé d'inhibition est un solvant organique.

**13.** Procédé selon l'une quelconque des revendications 1 à 11, dans lequel le composé d'inhibition est une solution aqueuse.

**14.** Procédé selon l'une quelconque des revendications 1 à 9, dans lequel le composé d'inhibition est ajouté par intermittence au cours du traitement de la source d'alimentation en hydrocarbure.

**15.** Procédé selon l'une quelconque des revendications 1 à 9, dans lequel le composé d'inhibition est ajouté en continu au cours du traitement de la source d'alimentation en hydrocarbure.

**16.** Procédé selon l'une quelconque des revendications 1 à 15, dans lequel la charge d'alimentation en hydrocarbure contient au moins une fraction choisie parmi le groupe constitué par:

a. l'éthane;
b. le propane;
c. le butane;
d. le solvant naphta;
e. le kérosène; et
f. le gasoil.

**17.** Procédé selon l'une quelconque des revendications 1 à 16, dans lequel une mince couche de coke inactive par voie catalytique se forme sur les surfaces entrant en contact avec la charge d'alimentation en hydrocarbure en aval de la section de chauffage à rayonnement.

**18.** Procédé selon l'une quelconque des revendications 1 à 17, dans lequel le composé d'inhibition est ajouté dans le domaine de 100 ppm à 10% sur base du débit massique de la charge d'alimentation en hydrocarbure au cours du traitement de la charge d'alimentation en hydrocarbure.

**19.** Procédé selon l'une quelconque des revendications 1 à 18, dans lequel, au cours de l'addition du composé d'inhibition, on maintient le four à une température se situant dans le domaine de 500 à 1200°C.

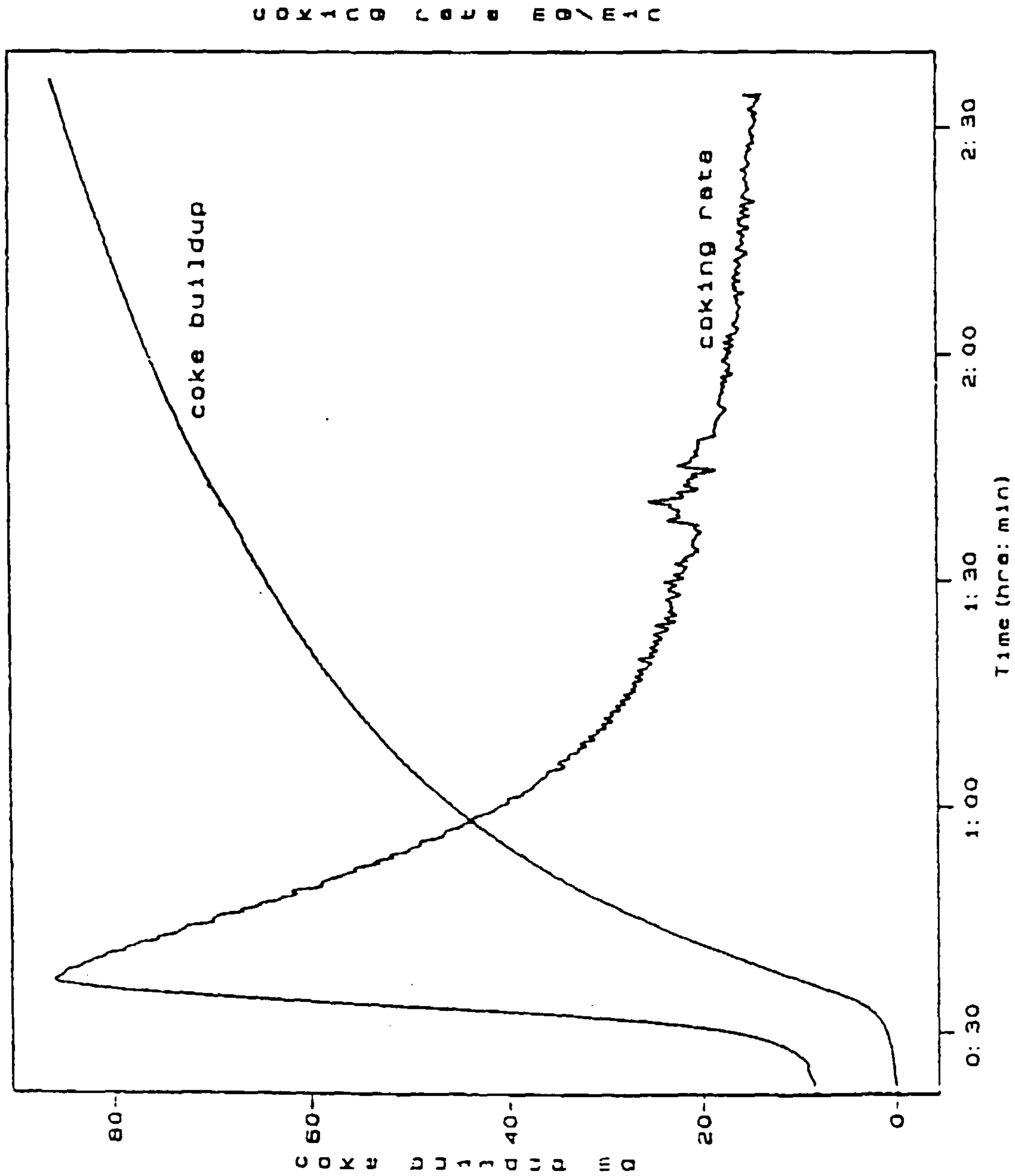
coke buildup
coking rate
Time (hrs: min)
0: 30
1: 00
1: 30
2: 00
2: 30
0
20
40
60
80

Fig. 1

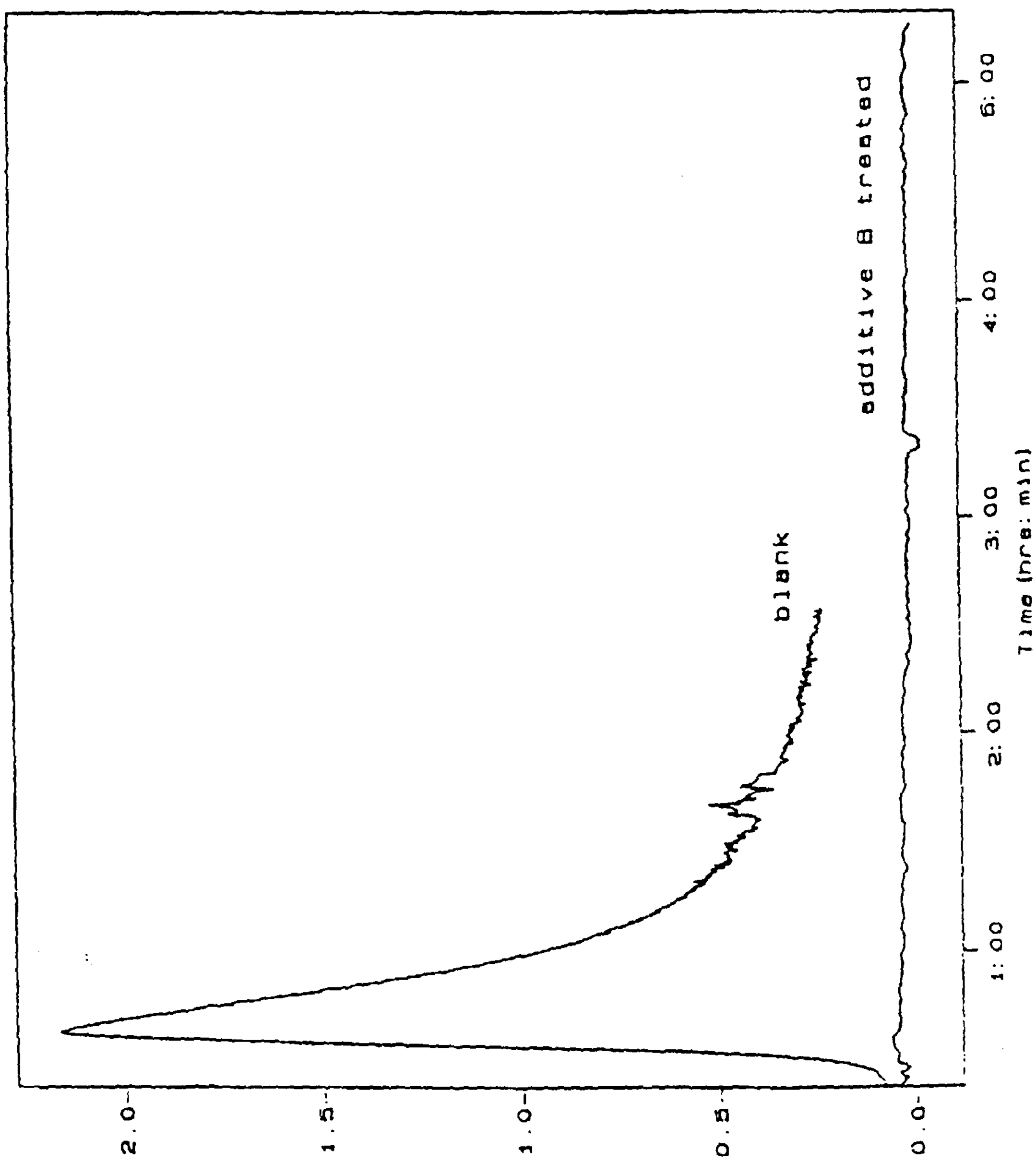
blank
additive B treated
Time (hrs: min)
1:00
2:00
3:00
4:00
6:00
0.0
0.5
1.0
1.5
2.0

Fig. 2